(19) Europäisches Patentamt
European Patent Office
Office européen des brevets

(11) **EP 4 729 106 A1**

(12) **EUROPEAN PATENT APPLICATION**
published in accordance with Art. 153(4) EPC

(43) Date of publication:
**22.04.2026 Bulletin 2026/17**

(21) Application number: **24823191.2**

(22) Date of filing: **22.05.2024**

(51) International Patent Classification (IPC):
**A61M 25/09** (2006.01)

(52) Cooperative Patent Classification (CPC):
**A61M 25/09**

(86) International application number:
**PCT/JP2024/018830**

(87) International publication number:
**WO 2024/257569 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.06.2023 JP 2023099120**

(71) Applicant: **ASAHI INTECC CO., LTD.
Seto-shi, Aichi, 489-0071 (JP)**

(72) Inventors:
• **NAGAO, Narumi**
**Seto-shi, Aichi 489-0071 (JP)**
• **CHIKAOKA, Sora**
**Seto-shi, Aichi 489-0071 (JP)**

(74) Representative: **Winter, Brandl - Partnerschaft
mbB
Alois-Steinecker-Straße 22
85354 Freising (DE)**

(54) **MEDICAL DEVICE**

(57) The object is to provide a guidewire that can prevent irreversible deformation at a distal end portion of a coil body and that can also prevent radial displacement of a wire constituting the coil body. A medical device 1 includes a core shaft 11, an outer coil body 21 formed by winding a wire w1, w1, and a distal end fixed portion 41 at which a distal end of the core shaft 11 and the outer coil body 21 are fixed to each other. The outer coil body 21 includes a loosely wound portion 21A with a gap between the adjacent wires w1 along the longitudinal axis direction of the core shaft 11, and the loosely wound portion 21A of the outer coil body 21 satisfies the following formulas (1) and (2): in the formulas (1) and (2), D represents the effective diameter of the outer coil body 21, d represents the wire diameter of the outer coil body 21, and Pa represents a pitch representative value of the wire w1 of the outer coil body 21.

$$3.5 \leq D/d \leq 6.5 \cdots (1)$$

$$1 < Pa \leq 3 \cdots (2)$$

FIG. 2

EP 4 729 106 A1

**Description**

[Technical field]

The present invention relates to a medical device.

[Background Art]

For example, when treating a lesion or the like occurring in a blood vessel, a guidewire is inserted prior to the insertion of a medical instrument such as a catheter to guide the instrument.
As such a guidewire, for example, one has been proposed in which a coil body is provided so as to cover the distal end portion of a reduced-diameter core shaft so that, for example, the guidewire can follow a complexly curved blood vessel and transmit rotational force applied at the proximal end to the distal end (see, e.g., Patent Literature 1).

[Citation List]

[Patent Literature]

[Patent Literature 1] JP 10-146390 A

[Summary of Invention]

[Technical Problem]

The distal end portion of the guidewire described above may be pre-shaped into a desired shape, enabling reliable insertion into a desired blood vessel among complexly curved blood vessels or branched blood vessels.
However, as the distal end portion of the guidewire is repeatedly shaped to accommodate the various blood vessel shapes, the coil body undergoes plastic deformation, which tends to result in permanent deformation in its shape or a radial displacement of part of the wire constituting the coil body.
When attempting to reshape such a guidewire in which the coil body has been plastically deformed, it may be difficult to reshape it into the desired shape, or part of the wire constituting the coil body may become displaced radially, thereby creating a step on the outer periphery of the guidewire.
The present invention has been made in light of the above circumstances, and its object is to provide a guidewire that can prevent irreversible deformation at a distal end portion of a coil body and that can also prevent radial displacement of a wire constituting the coil body.

[Solution to Problem]

Some aspects of the present disclosure provide the followings.

(1) A medical device including:

a core shaft;
an outer coil body that is formed by winding a wire, the outer coil body being disposed so as to cover at least a part of a distal end portion of the core shaft; and
a distal end fixed portion in which a distal end of the core shaft and a distal end of the outer coil body are fixed to each other, in which:

the outer coil body includes a loosely wound portion having a gap between the adjacent wires along a longitudinal axis direction of the core shaft; and
the loosely wound portion of the outer coil body satisfies the following formulas (1) and (2):

$$3.5 \leq D/d \leq 6.5 \cdots (1)$$

$$1 < Pa \leq 3 \cdots (2)$$

(in the formula (1), D represents an effective diameter of the outer coil body, and d represents a wire diameter of the outer coil body. In the formula (2), Pa represents a pitch representative value of the wire in the outer coil body.)

(2) The medical device according to the (1), in which the pitch representative value Pa satisfies 1<Pa≤2.

(3) The medical device according to the (2), in which the effective diameter D of the outer coil body and the wire diameter d of the outer coil body satisfy 4.5≤D/d≤5.

(4) The medical device according to the (2), in which the effective diameter D of the outer coil body and the wire diameter d of the outer coil body satisfy 5≤D/d≤5.5.

(5) The medical device according to any one of the (2) to (4), in which the pitch representative value Pa satisfies 1<Pa≤1.5.

(6) The medical device according to the (5), in which the pitch representative value Pa satisfies 1<Pa≤1.1.

(7) The medical device according to any one of the (1) to (6), in which a distal end of the loosely wound portion is located at the distal end of the outer coil body and a proximal end of the loosely wound portion is located at a position 10 mm to 30 mm from the distal end of the outer coil body toward a proximal end side.

(8) The medical device according to any one of the (1) to (7), further including an inner coil body that covers at least a part of the distal end portion of the core shaft, that is disposed inside the outer coil body, and that is formed by winding a wire at a constant pitch.

(9) The medical device according to the (8), in which a winding direction of the wire in the outer coil body and a winding direction of the wire in the inner coil body are opposite to each other, and an inclination angle of the wire in the outer coil body relative to a longitudinal axis of the core shaft is different from an inclination angle of the wire in the inner coil body relative to the longitudinal axis of the core shaft.

(10) The medical device according to any one of the (1) to (9), in which a bending angle of the distal end portion of the outer coil body relative to a distal end direction of the longitudinal axis is 180 degrees or less, the bending angle being measured such that a proximal end of the core shaft is twisted three times in a circumferential direction while the distal end fixed portion is fixed, and thereafter the fixing of the distal end fixed portion is released and the outer coil body is removed.

(11) The medical device according to the (10), in which the bending angle is 90 degrees or less.

(12) The medical device according to the (11), in which the bending angle is 45 degrees or less.

Note that, in the present specification, the term "effective diameter" is defined as a value obtained by the following expression: effective diameter = outer diameter of coil body - (radius of coil body wire × 2). The term "distal end side" refers to a direction along the longitudinal axis direction of the medical device (guidewire) toward the deeper (distal) part of the body cavity into which the device is inserted. The term "proximal end side" refers to a direction along the longitudinal axis direction of the medical device, opposite the "distal end side". The term "distal end" refers to an end portion on the distal end side of any member or portion, and the term "proximal end" refers to an end portion on the proximal end side of any member or portion. The term "distal end portion" refers to a part of any member or portion that includes the distal end thereof and extends from the distal end to the midpoint in the longitudinal axis direction toward the proximal end side. The term "proximal end portion" refers to a part of any member or portion that includes the proximal end thereof and extends from the proximal end to the midpoint in the longitudinal axis direction toward the distal end side. The term "radial direction" refers to a radial direction perpendicular to the longitudinal axis direction of the core shaft. The term "pitch representative value" refers to an index showing the degree of pitch of the wire constituting the outer coil body in the longitudinal axis direction of the core shaft.

[Advantageous Effects of Invention]

The present invention can provide the guidewire that can prevent the irreversible deformation at the distal end portion and that can also prevent the radial displacement of the wire constituting the coil body.

[Brief Description of Drawings]

FIG. 1 a schematic cross-sectional view illustrating a first embodiment.
FIG. 2 is a schematic cross-sectional view illustrating an enlarged part of FIG. 1.
FIG. 3 is a schematic cross-sectional view illustrating an enlarged part of a second embodiment.
FIG. 4A is an explanatory diagram illustrating a test method for residual deformation test.
FIG. 4B is a photograph showing the external appearance of an example of the residual deformation test.
FIG. 5 is an explanatory diagram illustrating a measurement method for the residual deformation test.

[Description of Embodiments]

A medical device of the present disclosure is a medical device that includes a core shaft, an outer coil body that is formed by winding a wire, the outer coil body being disposed so as to cover at least a part of a distal end portion of the core shaft, and a distal end fixed portion in which a distal end of the core shaft and a distal end of the outer coil body are fixed to each other. The outer coil body includes a loosely wound portion having a gap between the adjacent wires along a longitudinal axis direction of the core shaft. The loosely wound portion of the outer coil body satisfies the above formulas (1) and (2).

Hereinafter, first and second embodiments of the present invention are described with reference to the drawings. However, the present invention is not limited to the embodiments illustrated in the drawings. Further, the dimensions of each portion illustrated in the drawings are provided to facilitate understanding of the contents of the embodiments and do not necessarily correspond to the actual dimensions. Note that, in the first and second embodiments described below, a guidewire is described as an example of a medical device.

[First embodiment]

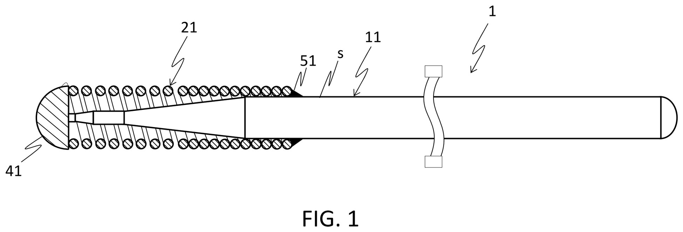

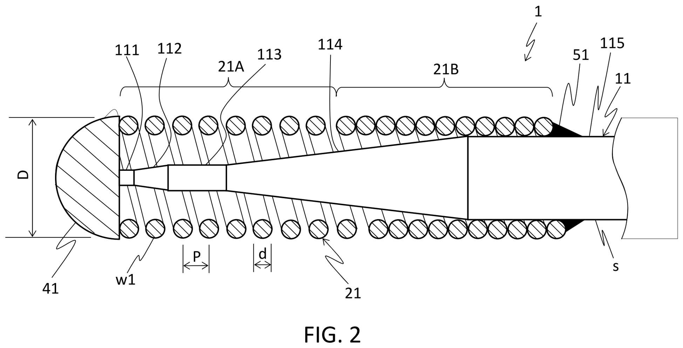

FIG. 1 and FIG. 2 are schematic cross-sectional views illustrating the first embodiment. As illustrated in FIG. 1 and FIG. 2, a guidewire 1 is generally composed of a core shaft 11, an outer coil body 21, a distal end fixed portion 41, and a proximal end fixed portion 51.

The core shaft 11 is an elongated member that constitutes the central axis of the guidewire 1. Specifically, for example, a distal end portion of the core shaft 11 can be formed to gradually reduce in diameter toward the distal end side.

The core shaft 11 of the present embodiment includes a first columnar portion 111, a first tapered portion 112, a second columnar portion 113, a second tapered portion 114, and a third columnar portion 115, in this order from the distal end. The first columnar portion 111 is a portion having a constant cross-sectional shape (e.g., a flat shape). The first tapered portion 112 is a tapered portion extending from the proximal end of the first columnar portion 111 toward the proximal end side. The second columnar portion 113 is a portion extending from the proximal end of the first tapered portion 112 toward the proximal end side and having a constant cross-sectional shape (e.g., a flat shape). The second tapered portion 114 is a tapered portion (e.g., a truncated cone shape) extending from the proximal end of the second columnar portion 113 toward the proximal end side. The third columnar portion 115 is a portion extending from the proximal end of the second tapered portion 114 toward the proximal end side and having a constant cross-sectional shape (e.g., a cylindrical shape). Note that at the boundaries between adjacent portions of the first columnar portion 111, first tapered portion 112, second columnar portion 113, second tapered portion 114, and third columnar portion 115, the outer shapes of the adjacent portions are configured to be identical and continuous.

As a material constituting the core shaft 11, for example, stainless steel such as SUS304, a superelastic alloy such as an Ni-Ti alloy, or the like can be used from the viewpoint of increasing the flexibility of the guidewire 1 and imparting antithrombogenicity and biocompatibility.

The outer coil body 21 is a spiral member that is formed by winding a wire w1 and that is disposed so as to cover at least a part of the distal end portion of the core shaft 11. As the wire w1, it is possible to use a single wire or multiple single wires, a single stranded wire or multiple stranded wires, or a combination thereof. Note that a single wire refers to one individual wire, while a stranded wire refers to a bundle of multiple individual wires that have been pre-twisted together.

The outer coil body 21 includes a loosely wound portion 21A having a gap between the adjacent wires w1, w1 along the longitudinal axis direction of the core shaft 11. The loosely wound portion 21A of the outer coil body 21 is configured to satisfy the following formulas (1) and (2).

$$3.5 \leq D/d \leq 6.5 \cdots (1)$$

$$1 < Pa \leq 3 \cdots (2)$$

In the formula (1), D represents the effective diameter of the outer coil body 21, and d represents the wire diameter of the outer coil body 21. In the formula (2), Pa represents the pitch representative value of the wire w1 in the outer coil body 21.

Specific examples of the pitch representative value Pa include an arithmetic mean value $(P_{min}+P_{max})/2d$ of the pitches of the outer coil body 21 in the loosely wound portion 21A, and the mean value $\Sigma P/n$ calculated from each pitch P. Note that $P_{min}$ represents the minimum pitch among the pitches P of the outer coil body 21, $P_{max}$ represents the maximum pitch among the pitches P of the outer coil body 21, d represents the wire diameter of the outer coil body 21, P represents the pitch in the outer coil body 21, and n represents the number of turns of the wire w1 in the loosely wound portion 21A.

Here, the pitch representative value Pa preferably satisfies $1 < Pa \leq 2$, more preferably $1 < Pa \leq 1.5$, even more

preferably $1 < Pa \leq 1.1$. Further, the lower limit of the pitch representative value Pa is preferably 1.01 ($1.01 \leq Pa$), regardless of its upper limit. When the pitch representative value Pa satisfies the above relationship, it becomes possible to effectively prevent the irreversible deformation at the distal end portion of the outer coil body 21 and the radial displacement of the wire w1 constituting the outer coil body 21.

The effective diameter D of the outer coil body 21 and the wire diameter d of the outer coil body 21 preferably satisfy $4.5 \leq D/d \leq 5$, and also preferably satisfy $5 \leq D/d \leq 5.5$. Further, the effective diameter D of the outer coil body 21 and the wire diameter d of the outer coil body 21 preferably satisfy $5.5 \leq D/d \leq 6.5$. When D/d satisfies at least one of the above relationships, it is possible to prevent both the irreversible deformation at the distal end portion of the outer coil body 21 and an occurrence of the positional displacement.

Note that, in the present embodiment, the distal end of the loosely wound portion 21A is located at the distal end of the outer coil body 21, and the proximal end of the loosely wound portion 21A is located at a position 10 mm to 30 mm from the distal end of the outer coil body 21 toward the proximal end side. In other words, the loosely wound portion 21A is located in a specific range at the distal end portion of the outer coil body 21. This can effectively prevent the residual deformation of the outer coil body 21 and the positional displacement of the wire w1 at the distal end portion of the guidewire 1 to be shaped, thereby allowing the guidewire to be repeatedly shaped (reshaped) into a desired shape.

As a material constituting the outer coil body 21, for example, stainless steel such as SUS316, a superelastic alloy such as an Ni-Ti alloy, radiopaque metal such as platinum or tungsten, or the like can be used.

The distal end fixed portion 41 is a portion where the distal end of the core shaft 11 and the distal end of the outer coil body 21 are fixed to each other. As the shape of the distal end fixed portion 41, the distal end side may be formed into a smoothly curved hemispherical shape so as not to damage the inner wall of the blood vessel when the guidewire 1 advances in the blood vessel.

Specifically, the distal end fixed portion 41 can be formed, for example, by integrally brazing the distal end of the core shaft 11 and the distal end of the outer coil body 21 via a brazing material using a brazing material, or by melt-molding a part that is originally the distal end portion of the core shaft 11 and a part that is originally the distal end portion of the outer coil body 21. Examples of the brazing material that can be used for brazing include a metal brazing material such as an Sn-Pb alloy, a Pb-Ag alloy, an SnAg alloy, or an Au-Sn alloy.

The proximal end fixed portion 51 is a portion where an outer peripheral surface s of the core shaft 11 and the proximal end portion of the outer coil body 21 are fixed to each other. The proximal end fixed portion 51 may be provided at any position on the outer peripheral surface s of the core shaft 11. In the present embodiment, the proximal end fixed portion 51 is provided on the outer peripheral surface s of the third columnar portion 115.

Specifically, the proximal end fixed portion 51 can be formed, for example, by brazing the outer peripheral surface s of the core shaft 11 and the proximal end portion of the outer coil body 21 via a brazing material or by bonding them using an adhesive. Examples of the brazing material include the brazing material used to form the distal end fixed portion 41. Examples of the adhesive include an epoxy adhesive, a cyanoacrylate adhesive, and an acrylic adhesive.

Next, the usage mode of the guidewire 1 is described. First, before inserting the guidewire 1 into a blood vessel, the distal end portion of the guidewire 1 is bent (shaped) into a desired shape (e.g., a J-shape). Then, the guidewire 1 is inserted into the blood vessel from its distal end, and by manipulating the proximal end portion of the guidewire 1 that remains exposed outside the body, the distal end portion is advanced to the site to be treated within the blood vessel.

Next, after the distal end portion of the guidewire 1 has reached the site to be treated, the proximal end of the guidewire 1 is inserted into the lumen of a medical instrument (not illustrated) such as a catheter from its distal end, and the medical instrument is advanced into the blood vessel along the guidewire 1. Next, after the medical instrument has reached the site to be treated, various treatments are performed using the medical instrument. Next, after the treatment is completed, the medical instrument is pulled out of the body along the guidewire 1, and the guidewire 1 is removed from the blood vessel.

It should be noted that, through the use of the guidewire 1 described above, the distal end portion of the removed guidewire 1 tends to deform into a shape different from the initially formed shape. Thus, when another medical instrument is subsequently used or when the same medical instrument is inserted into the blood vessel again, the distal end portion of the removed guidewire 1 may be shaped again into a desired shape (which may be a new shape different from the initially formed shape) and used (reshaped). The reshaped guidewire 1 can be reused, for example, in the same manner as in the use procedure for the guidewire 1 described above.

As described above, the guidewire 1 (medical device) has the above-described configuration, making it possible to prevent the irreversible deformation at the distal end portion of the outer coil body 21 and the radial displacement of the wire w1 constituting the outer coil body 21. As a result, even if the distal end portion of the guidewire 1 is repeatedly shaped, the outer coil body 21 is less likely to form residual deformation, and when re-shaping is performed, it can be easily and reliably shaped into a desired shape. Further, since a step is less likely to be created on the outer coil body 21, the guidewire 1 and the medical instrument such as a catheter used in combination with the guidewire 1 can be delivered more smoothly to the treatment site.

It is presumed that the prevention of the irreversible deformation and the prevention of the positional displacement of the wire described above are due to the following reasons.

That is, if the wire diameter d is large relative to the effective diameter D of the outer coil body, the relative shear force in the transverse direction of the wire increases, making the outer coil body more prone to form residual deformation due to the accumulation of internal stress. On the other hand, if the wire diameter d is small relative to the effective diameter D of the outer coil body, the wire tends to move more easily in the radial direction, making the positional displacement more likely to occur.

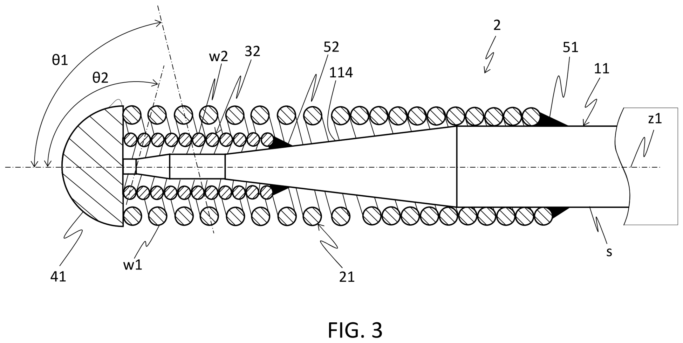

Further, when the pitch representative value Pa of the wire in the outer coil body is increased, the inherent flexibility of the coil body is improved, making it less likely for plastic deformation to occur when the outer coil body is bent (shaped). When the pitch representative value Pa of the wire in the outer coil body is increased, the degree of freedom in the axial direction of a longitudinal axis Z1 (see FIG. 3) of the outer coil body increases, making it less likely for the wire of the outer coil body to move radially and become positionally displaced. On the other hand, when the pitch representative value Pa of the wire in the outer coil body is reduced, the space in which the wire can exist becomes smaller, and as a result, the outer coil body become more likely to form residual deformation due to the accumulation of internal stress that occurs when the outer coil body is bent, and the wire is more likely to move radially and become positionally displaced.

Thus, it is presumed that specifically forming the guidewire 1 to satisfy the relationship of the above-mentioned formulas (1) and (2) makes it possible to prevent the irreversible deformation at the distal end portion of the outer coil body 21 while also preventing the radial displacement of the wire w1 constituting the outer coil body 21.

[Second embodiment]

FIG. 3 is a schematic cross-sectional view illustrating a second embodiment. As illustrated in FIG. 3, a guidewire 2 is generally composed of the core shaft 11, the outer coil body 21, an inner coil body 32, the distal end fixed portion 41, and proximal end fixed portions 51 and 52. The guidewire 2 differs from the first embodiment in that it further includes the inner coil body 32. Note that the configuration of the second embodiment is the same as that of the first embodiment, except for the configuration of the inner coil body 32 described below. Thus, identical portions are designated by the same reference symbols and detailed description thereof is omitted. Further, the usage mode of the guidewire 2 is the same as that of the first embodiment.

The inner coil body 32 is a spiral member that covers at least a part of the distal end portion of the core shaft 11, that is disposed inside the outer coil body 21, and that is formed by winding a wire w2 at a constant pitch. As the wire w2, it is possible to use, for example, a single wire or multiple single wires, a stranded wire or multiple stranded wires, or a combination thereof.

As a material constituting the inner coil body 32, for example, stainless steel such as SUS316, a superelastic alloy such as an Ni-Ti alloy, radiopaque metal such as platinum or tungsten, or the like can be used.

The inner coil body 32 can have, for example, its distal end integrally fixed to the core shaft 11 and the outer coil body 21 at the distal end fixed portion 41, and its proximal end fixed to anywhere on the outer peripheral surface s of the core shaft 11. The inner coil body 32 of the present embodiment has its distal end fixed at the distal end fixed portion 41 and its proximal end fixed on the outer peripheral surface s of the second tapered portion 114 of the core shaft 11. As a method for fixing the inner coil body 32 at the distal end fixed portion 41 and the proximal end fixed portion 52, for example, a method similar to the fixing method at the distal end fixed portion 41 and the proximal end fixed portion 51 of the outer coil body 21 described above, or the like, can be used.

The winding direction of the wire w1 in the outer coil body 21 and the winding direction of the wire w2 in the inner coil body 32 may be opposite to each other, and an inclination angle $\theta 1$ of the wire w1 in the outer coil body 21 relative to the longitudinal axis z1 of the core shaft 11 may be different from an inclination angle $\theta 2$ of the wire w2 in the inner coil body 32 relative to the longitudinal axis z1 of the core shaft 11 (see FIG. 3).

That is, one of the outer coil body 21 and the inner coil body 32 may be S-twisted and the other Z-twisted, and the winding angle $\theta 1$ of the outer coil body 21 relative to the distal end direction of the longitudinal axis z1 of the core shaft 11 may be different from the winding angle $\theta 2$ of the inner coil body 32 relative to the distal end direction of the longitudinal axis z1 of the core shaft 11. This makes it possible to more effectively prevent the wire of one coil body (e.g., the outer coil body 21) from getting into a gap between the adjacent wires of the other coil body (e.g., the inner coil body 32).

As described above, the guidewire 2 (medical device) includes the inner coil body 32, and thus, it is possible to prevent the wire w1 constituting the outer coil body 21 from being displaced in the radial direction.

It is to be understood that the present disclosure is not limited to the configurations of the embodiments described above, but rather is defined by the scope of the claims, and is intended to include all modifications within the meaning and scope equivalent to the claims. The configurations of the above-described embodiments may be partially omitted, replaced with other configurations, or supplemented with additional configurations.

For example, in the above-described embodiment, the guidewire 1 (medical device) has been described in which the outer coil body 21 includes the loosely wound portion 21A and a densely wound portion 21B. However, the outer coil body 21 may be configured only with the loosely wound portion 21A without having the densely wound portion 21B.

Further, in the above-described embodiments, the guide wires 1 and 2 (medical devices) have been described in which the loosely wound portion 21A is located at the distal end portion of the outer coil body 21. However, the loosely wound portion 21A may be located midway along the longitudinal axis direction of the outer coil body or at the proximal end portion.

Further, in the above-described embodiments, the guidewires 1 and 2 (medical devices) have been described in which the loosely wound portion 21A is provided at only one location on the outer coil body 21. However, the loosely wound portion 21A may be provided at two or more independent locations on the outer coil body.

Further, in the above-described embodiments, the guidewires 1 and 2 have been described in which the core shaft 11 is configured in this order from the distal end: the first columnar portion 111, the first tapered portion 112, the second columnar portion 113, the second tapered portion 114, and the third columnar portion 115. However, the shape of the core shaft is not particularly limited. For example, the core shaft may not include a tapered portion or may be configured only with a tapered portion.

<Experimental results>

The specifications of the medical devices (guidewires) used for evaluation are shown in Table 1. Further, other specifications are as follows. Note that the pitch representative value $P_a$ for each sample was calculated using a formula $P_a = (P_{min} + P_{max})/2d$, where $P_{min}$ represents the minimum pitch of the outer coil body, $P_{max}$ represents the maximum pitch of the outer coil body, and d represents the wire diameter of the outer coil body. Further, the distal end of the loosely wound portion of each sample is located at the distal end of the outer coil body, and the length of the loosely wound portion in Table 1 indicates the length from the distal end to the proximal end of the loosely wound portion.

[Outer coil body]

·Material: Pt-Ni alloy
·Coil effective diameter D: 0.36 mm - (d/2 × 2)
·Coil outer diameter: 0.36 mm
·Wire diameter d: see Table 1
·Pitch representative value $P_a$: see Table 1
·Length of loosely wound portion: see Table 1

[Inner coil body]

·Material: stainless steel
·Coil outer diameter: 0.2 mm
·Wire diameter: 0.025 mm

<Evaluation>

The guidewires shown in Table 1 were evaluated for their ability to prevent residual deformation and positional displacement according to the following methods. The results are also shown in Table 1.

[Residual deformation prevention]

While fixing the distal end fixed portion of the guidewire to be used in the test (Residual deformation test), the proximal end of the core shaft was twisted three times in the circumferential direction, after which the fixing of the distal end fixed portion was released, and the outer coil body was removed and measured. At this time, the degree of bending (bending angle) of the distal end portion of the outer coil body relative to the distal end direction of the longitudinal axis was measured, and this measurement value was used as an index to evaluate the residual deformation prevention.

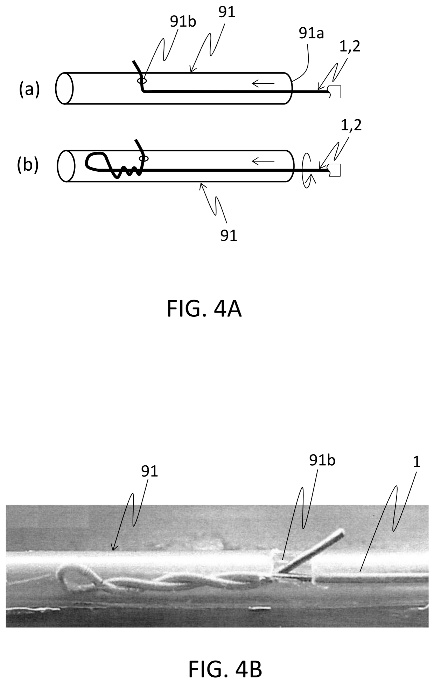

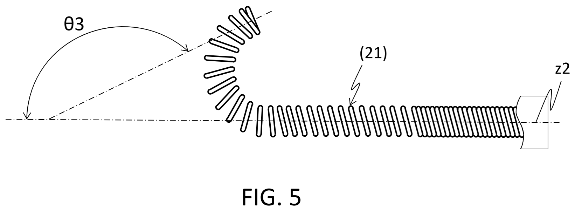

Specifically, as illustrated in FIG. 4A(a), the guidewire to be tested was inserted into one opening 91a of a transparent silicone tube 91 with an inner diameter of 1.25 mm, and the distal end portion of the guidewire was fixed to the silicone tube 91 via a hole 91b. Next, as illustrated in FIG. 4A(b), the guidewire was twisted three times in the circumferential direction relative to the silicone tube 91. FIG. 4B is a photograph showing the external appearance of an example of the guidewire after being twisted three times in the circumferential direction. Next, the fixing of the distal end of the guidewire was released, and the guidewire was removed from the silicone tube 91. Next, the guidewire was disassembled to remove the outer coil body. Then, as illustrated in FIG. 5, the angle (bending angle θ3) at which the distal end portion of the outer coil body (21) was bent relative to a longitudinal axis z2 of the outer coil body (21) (the same as the axis in the longitudinal direction of the outer coil body before the test) was measured.

In this case, if the bending angle θ3 is 180 degrees or less, the residual deformation prevention is evaluated as good, and if the bending angle θ3 is greater than 180 degrees, the residual deformation prevention is evaluated as poor. Further, if the bending angle θ3 is 90 degrees or less, the residual deformation prevention is evaluated as even better, and if the bending angle θ3 is 45 degrees or less, the residual deformation prevention was evaluated as best.

[Displacement prevention]

After the evaluation of the residual deformation prevention described above, the outer coil body was observed under a microscope at 20-hold magnification. At that time, the number of displacement sites (sites clearly recognized as being displaced) per single outer coil body was counted, and this number was used as an index to evaluate the displacement prevention.

[Table 1]

| Sample No. | Wire diameter of outer coil body d [μm] | Effective diameter of outer coil body D [mm] | D/d [-] | Minimum pitch Pmin [μm] | Maximum pitch Pmax [μm] | Pitch representative value Pa [-] | Loosely wound portion length [mm] | Inner coil body | Bending angle [degrees] | Average number of displacement sites of outer coil body wire [sites/outer coil body] |
|---|---|---|---|---|---|---|---|---|---|---|
| 1 | 65 | 0.30 | 4.5 | 65 | 77 | 1.09 | 15 | Yes | 68 | 0.67 |
| 2 | 65 | 0.30 | 4.5 | 65 | 77 | 1.09 | 15 | No | 68-94 | 0.33 |
| 3 | 65 | 0.30 | 4.5 | 65 | 77 | 1.09 | 15 | Yes | 94 | 0.67 |
| 4 | 65 | 0.30 | 4.5 | 88 | 101 | 1.45 | 30 | Yes | 70 | ≤ 0.33 |
| 5 | 65 | 0.30 | 4.5 | 88 | 101 | 1.45 | 30 | Yes | 117 | ≤ 0.33 |
| 6 | 65 | 0.30 | 4.5 | 113 | 124 | 1.82 | 30 | Yes | 158 | ≤ 0.33 |
| 7 | 65 | 0.30 | 4.5 | 113 | 124 | 1.82 | 30 | Yes | 170 | ≤ 0.33 |
| 8 | 60 | 0.30 | 5.0 | 60 | 71 | 1.09 | 15 | Yes | 47 | 0.33 |
| 9 | 60 | 0.30 | 5.0 | 60 | 71 | 1.09 | 15 | Yes | 52 | 0.33 |
| 10 | 60 | 0.30 | 50 | 82 | 93 | 1.46 | 30 | Yes | 27 | ≤ 0.33 |
| 11 | 60 | 0.30 | 5.0 | 82 | 93 | 1.46 | 30 | Yes | 35 | ≤ 0.33 |
| 12 | 60 | 0.30 | 5.0 | 104 | 115 | 1.83 | 30 | Yes | 98 | ≤ 0.33 |
| 13 | 60 | 0.30 | 50 | 104 | 115 | 1.83 | 30 | Yes | 172 | < 0.33 |
| 14 | 55 | 0.31 | 5.5 | 55 | 55 | 1.01 | 30 | Yes | 10-33 | 0.33 |
| 15 | 55 | 0.31 | 5.5 | 55 | 55 | 1.02 | 30 | Yes | 10-29 | 0.33 |
| 16 | 55 | 0.31 | 5.5 | 55 | 65 | 1.09 | 30 | Yes | 10 | 0.33 |
| 17 | 55 | 0.31 | 5.5 | 55 | 65 | 1.09 | 15 | Yes | 14 | 0.33 |
| 18 | 55 | 0.31 | 5.5 | 55 | 65 | 1.09 | 30 | Yes | 18 | 0.33 |
| 19 | 55 | 0.31 | 5.5 | 55 | 65 | 1.09 | 15 | Yes | 29 | 0.33 |
| 20 | 55 | 0.31 | 5.5 | 55 | 65 | 1.09 | 15 | No | 10-29 | 3.00 |
| 21 | 55 | 0.31 | 5.5 | 75 | 85 | 1.45 | 30 | Yes | 0 | ≤ 0.33 |
| 22 | 55 | 0.31 | 5.5 | 75 | 85 | 1.45 | 30 | Yes | 6 | < 0.33 |
| 23 | 55 | 031 | 5.5 | 75 | 85 | 1.45 | 30 | Yes | 10 | ≤ 0.33 |
| 24 | 55 | 0.31 | 5.5 | 75 | 85 | 1.45 | 30 | Yes | 13 | < 0.33 |

(continued)

| Sample No. | Wire diameter of outer coil body d[μm] | Effective diameter of outer coil body D[mm] | D/d [-] | Minimum pitch Pmin [μm] | Maximum pitch Pmax [μm] | Pitch representative value Pa [-] | Loosely wound portion length [mm] | Inner coil body | Bending angle [degrees] | Average number of displacement sites of outer coil body wire [sites/outer coil body] |
|---|---|---|---|---|---|---|---|---|---|---|
| 25 | 55 | 0.31 | 5.5 | 75 | 85 | 1.45 | 30 | Yes | 21 | ≤ 0.33 |
| 26 | 55 | 0.31 | 5.5 | 95 | 105 | 1.82 | 30 | Yes | 43 | < 0.33 |
| 27 | 55 | 0.31 | 5.5 | 95 | 105 | 1.82 | 30 | Yes | 55 | ≤ 0.33 |
| 28 | 55 | 0.31 | 5.5 | 150 | 160 | 282 | 30 | Yes | 158 | ≤ 0.33 |
| 29 | 55 | 0.31 | 5.5 | 150 | 160 | 2.82 | 30 | Yes | 201 | ≤ 0.33 |
| 30 | 55 | 0.31 | 5.5 | 55 | 55 | 1.00 | 0 | Yes | 17 | 2.00 |
| 31 | 55 | 0.31 | 5.5 | 55 | 55 | 1.00 | 0 | Yes | 33 | 2.00 |
| 32 | 55 | 0.31 | 5.5 | 55 | 55 | 1.00 | 0 | No | 17-33 | 7.00 |

As can be seen from the results in Table 1, for No. 1 to 29, the pitch representative values Pa were greater than 1, and thus the displacement prevention was good. The ratio, the effective diameter D of the outer coil body 21/the wire diameter d of the outer coil body 21, is preferably 4.5 or more and 5.5 or less. The ratio, the effective diameter D of the outer coil body 21/the wire diameter d of the outer coil body 21, is preferably 4.5 or more and 5 or less. This is because a smaller effective diameter D makes it easier to apply the outer coil body to a guidewire with a small maximum outer diameter. Further, the ratio, the effective diameter D of the outer coil body 21/the wire diameter d of the outer coil body 21, is also preferably 5 or more and 5.5 or less. This is because the bending angle is thereby reduced.

[Reference Signs List]

1, 2 Medical device (guidewire)
11 Core shaft
21 Outer coil body
21A Loosely wound portion
32 Inner coil body
41 Distal end fixed portion
D Effective diameter of outer coil body
d Wire diameter of outer coil body
Pa Pitch representative value
$\theta1$, $\theta2$ Inclination angle
$\theta3$ Bending angle
z2 Longitudinal axis of outer coil body

**Claims**

1. A medical device comprising:

    a core shaft;
    an outer coil body that is formed by winding a wire, the outer coil body being disposed so as to cover at least a part of a distal end portion of the core shaft; and
    a distal end fixed portion in which a distal end of the core shaft and a distal end of the outer coil body are fixed to each other, wherein:

    the outer coil body includes a loosely wound portion having a gap between adjacent wires along a longitudinal axis direction of the core shaft; and
    the loosely wound portion of the outer coil body satisfies following formulas (1) and (2):

$$3.5 \leq D/d \leq 6.5 \cdots (1)$$

$$1 < Pa \leq 3 \cdots (2)$$

    (in the formula (1), D represents an effective diameter of the outer coil body, and d represents a wire diameter of the outer coil body. In the formula (2), Pa represents a pitch representative value of the wire in the outer coil body.)

2. The medical device according to claim 1, wherein the pitch representative value Pa satisfies $1 < Pa \leq 2$.

3. The medical device according to claim 2, wherein the effective diameter D of the outer coil body and the wire diameter d of the outer coil body satisfy $4.5 \leq D/d \leq 5$.

4. The medical device according to claim 2, wherein the effective diameter D of the outer coil body and the wire diameter d of the outer coil body satisfy $5 \leq D/d \leq 5.5$.

5. The medical device according to any one of claims 2 to 4, wherein the pitch representative value Pa satisfies 1<Pa≤1.5.

6. The medical device according to claim 5, wherein the pitch representative value Pa satisfies 1<Pa≤1.1.

7. The medical device according to any one of claims 1 to 6, wherein a distal end of the loosely wound portion is located at the distal end of the outer coil body and a proximal end of the loosely wound portion is located at a position 10 mm to 30 mm from the distal end of the outer coil body toward a proximal end side.

8. The medical device according to any one of claims 1 to 7, further comprising an inner coil body that covers at least a part of the distal end portion of the core shaft, that is disposed inside the outer coil body, and that is formed by winding a wire at a constant pitch.

9. The medical device according to claim 8, wherein a winding direction of the wire in the outer coil body and a winding direction of the wire in the inner coil body are opposite to each other, and an inclination angle of the wire in the outer coil body relative to a longitudinal axis of the core shaft is different from an inclination angle of the wire in the inner coil body relative to the longitudinal axis of the core shaft.

10. The medical device according to any one of claims 1 to 9, wherein a bending angle of the distal end portion of the outer coil body relative to a distal end direction of the longitudinal axis is 180 degrees or less, the bending angle being measured such that a proximal end of the core shaft is twisted three times in a circumferential direction while the distal end fixed portion is fixed, and thereafter the fixing of the distal end fixed portion is released and the outer coil body is removed.

11. The medical device according to claim 10, wherein the bending angle is 90 degrees or less.

12. The medical device according to claim 11, wherein the bending angle is 45 degrees or less.

FIG. 1

FIG. 2

FIG. 3

FIG. 4A

FIG. 4B

FIG. 5

## INTERNATIONAL SEARCH REPORT

International application No.

**PCT/JP2024/018830**

| A. | CLASSIFICATION OF SUBJECT MATTER |
|---|---|

*A61M 25/09*(2006.01)i
FI: A61M25/09 516

According to International Patent Classification (IPC) or to both national classification and IPC

| B. | FIELDS SEARCHED |
|---|---|

Minimum documentation searched (classification system followed by classification symbols)

A61M25/09

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2016-2210 A (FMD CO., LTD.) 12 January 2016 (2016-01-12) paragraphs [0023]-[0028], fig. 1 | 1-7 |
| Y | | 8-12 |
| Y | JP 2017-164200 A (TERUMO KABUSHIKI KAISHA) 21 September 2017 (2017-09-21) paragraph [0069], fig. 2 | 8-12 |
| Y | JP 2009-511184 A (BOSTON SCIENTIFIC LIMITED) 19 March 2009 (2009-03-19) fig. 10 | 8-12 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 August 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

International application No.

**PCT/JP2024/018830**

| Patent document cited in search report | | | Publication date (day/month/year) | Patent family member(s) | | | Publication date (day/month/year) |
|---|---|---|---|---|---|---|---|
| JP | 2016-2210 | A | 12 January 2016 | JP | 5735159 | B1 | |
| JP | 2017-164200 | A | 21 September 2017 | (Family: none) | | | |
| JP | 2009-511184 | A | 19 March 2009 | US fig. 10 WO | 2007/0083132 2007/044783 | A1 A1 | |

Form PCT/ISA/210 (patent family annex) (July 2022)

**EP 4 729 106 A1**

## REFERENCES CITED IN THE DESCRIPTION

**Patent documents cited in the description**

- JP 10146390 A